# NEUE EUROPÄISCHE PATENTSCHRIFT

(11) **EP 0 685 454 B2**
(45) Veröffentlichungstag und Bekanntmachung des Hinweises auf die Entscheidung über den Einspruch: **27.06.2001**
(45) Hinweis auf die Patenterteilung: 15.10.1997
(21) Anmeldenummer: 95250125.2
(22) Anmeldetag: 24.05.1995
(51) Int. Cl.: C07C 69/76, A61K 6/083, C07C 233/54, C08F 220/30

(54) **Röntgenopake Dentalmaterialien**
X-ray opaque dental materials
Matériaux dentaires opaques aux rayons X

(30) Priorität: 30.05.1994 DE 4419386
(43) Veröffentlichungstag der Anmeldung: 06.12.1995
(73) Patentinhaber: Ivoclar Vivadent AG, 9494 Schaan (LI)
(72) Erfinder: Rheinberger, Volker, Dr., FL-9490 Vaduz (LI); Moszner, Norbert, Prof. Dr., FL-9492 Eschen (LI); Salz, Ulrich, Dr., D-88138 Weissensberg (DE)
(74) Vertreter: UEXKÜLL & STOLBERG

(56) Entgegenhaltungen:
- EP-A- 0 684 222
- DE-A- 1 492 183
- DE-A- 3 521 721
- US-A- 3 346 620
- US-A- 3 361 700
- Supplement to Diagnostic Imaging Europe, June 1995, P. Dawson, "Design plays major role in new contrast media", S. CM3, CM4, CM40

## Beschreibung

Die Erfindung betrifft röntgenopake Ester und Amide iodsubstituierter Benzoesäure gemäß Formel I sowie daraus hergestellte Polymere und Dentalmaterialien.

In der DE-OS 24 58 380 werden röntgenopake Zahnfüllmassen auf Polyesterharzbasis beschrieben. Die Röntgenopazität wird durch Zusatz röntgenopaker Füllstoffe erzielt. Als Füllstoffe werden Gläser, die Oxide oder Carbonate des Lanthans, Strontiums, Tantals oder Hafniums enthalten, eingesetzt.

Die DE-OS 24 20 351 betrifft fließbare, hydrophile Zahnfüllmaterialien für die Wurzelbehandlung, die als Röntgenstrahlen undurchlässige Füllstoffe Bariumsulfat, Tantal, lodalphionsäure, lopanoinsäure, Ipodoinsäure oder Wismutsubcarbonat enthalten. Als polymerisationsfähiges Monomer wird bevorzugt Hydroxyethylmethacrylat verwendet.

In der EP-PS 0 011 735 werden als röntgenopake Füllstoffe feste, schwerlösliche Schwermetallverbindungen, wie Bariumsulfat, Bariumfluorid und Bariumsilikat, Wismut-, Zirkon-, Lanthan- und Thoriumverbindungen sowie Verbindungen der Seltenen Erdmetalle offenbart. Darüber hinaus wird die Verwendung anorganischer und organischer lodverbindungen als Röntgenkontrastmittel erwähnt.

Aus der EP-PS 0 189 540 sind Dentalwerkstoffe bekannt, die zur Erzielung von Röntgenopazität Fluoride der Seltenen Erdmetalle enthalten. Diese Fluoride werden im allgemeinen als Pulver in den Dentalwerkstoff eingearbeitet. Bevorzugte Verbindung ist Ytterbiumfluorid.

In den oben genannten Beispielen wird die Röntgenopazität der Dentalwerkstoffe durch röntgenopake Füllmaterialien erzielt. Nachteilig an diesen Materialien ist, daß die häufig eingesetzten Schwermetalle toxisch und teilweise radioaktiv sind, während die Oxide der Seltenen Erden zu unerwünschten Verfärbungen der Füllung oder Prothese führen können. Die Transparenz der bekannten röntgenopaken mikrogefüllten Dentalwerkstoffe ist unbefriedigend und die Hochglanzpolierbarkeit nicht ausreichend. Da die Röntgenopazität in allen Fällen über den Füllstoff realisiert wird, ist die Herstellung wenig oder nicht-gefüllter röntgenopaker Dentalmaterialien, zum Beispiel für Befestigungszemente oder Bondings, auf diesem Wege nicht möglich.

Röntgenopake Dentalmaterialien deren Röngenopazität nicht an den Füllstoff gebunden sind, werden in der DE-OS 21 21 480 offenbart. Diese Offenlegungsschrift betrifft Methacrylatteilchen in Perlform, die aliphatische Halogenide zur Erzielung von Röntgenopazität enthalten. Die Perlpoymerisate sind in monomeren Alkylmethacrylaten löslich und werden durch Suspensionspolymerisation in Gegenwart der aliphatischen Halogenverbindungen hergestellt. Als Halogenverbindungen werden brom- oder iodhaltige Derivate beschrieben, wobei sich iodhaltige Verbindungen ausschließlich in Kombination mit bromhaltigen Substanzen eignen. In einer bevorzugten Ausführungsform werden die Perlpolymerisate zur Verstärkung der Röntgenopazität oberflächlich mit Schwermetallverbindungen angefärbt. Für die erhaltenen Materialien ist charakteristisch, daß ein chemischer Einbau von halogenhaltigen Endgruppen in das Polymer durch Kettenübertragung bzw. Kettenabbruch erfolgt und somit vergleichsweise nur ein relativ geringer Halogengehalt im Polymer (unter 18 Gew. %) erreichbar ist. Im Falle von Bromverbindungen würde dieser Halogengehalt nur zu schwachen röntgenopaken Eigenschaften führen. Darüberhinaus wird durch die Kettenübertragung der Polymerisationsgrad der Polymerisate entsprechend verringert, d.h.. beim Einsatz einer hohen Konzentration einer Halogenverbindung kann der für die Materialeigenschaften der Polymerisate notwendige Polymerisationsgrad nicht erreicht werden. Schießlich ist bei einem physikalischen Einbau die Halogenverbindung mit organischen Lösungsmitteln auswaschbar.

Darüber hinaus wurden Materialien beschrieben, die schwermetallorganische Verbindungen enthalten, wie zum Beispiel Triphenylwismut (Y. Delaviz, Z. X. Zhang, I. Cabasso, J. Smith, *Polym*. *Prepr*. (Amer. Chem. Soc., Polym. Div.) **30** (1989) 215). Diese Materialien zeigen den Nachteil, daß die schwermetallorganischen Verbindungen leicht aus der polymeren Matrix ausgewaschen werden.

Der Einsatz von Schwermetallionen enthaltenden röntgenopaken Monomeren, wie Zink- oder Bariumacrylat, führt zu Materialien, die im Vergleich zu den unmodifizierten Harzen deutlich verschlechterte mechanische Eigenschaften aufweisen (K W. M. Davey, B. E. Causton, *J*. *Dent*. **10** (1982) 254).

In der WO-82/01006 werden röntgenopake Homo- und Copolymere auf der Basis von Methacrylsäureestern beschrieben. Die Röntgenopazität wird durch kovalente Verknüpfung der Acrylsäurereste mit Röntgenstrahlen absorbierenden Atomen erzielt. Hierzu eignen sich besonders Halogenatome wie Chlor. Brom und Iod, wobei Brom bevorzugt wird da Chlor wenig effectiv und Iodsubstituierte Polymere zu instabil sind. Die Polymere werden in Form von Perlen, Krümmeln, Scheiben, Staben, Blöcken oder anderen Formen erhalten. Dabei werden ausschließlich Polymere aber keine Monomere auf der Basis von aliphatisch gebundenem Halogen beschrieben, wie beispielsweise Poly(2,3-dibrompropylmethacrylat), das durch aufwendige anionische Polymerisation von Allylmethacrylat und nachfolgende Bromierung erhalten wird Röntgenopake Polymere mit aromatisch gebundenem Halogen, das stabiler ist, sind auf diesem Wege nicht zugänglich.

Röntgenopake, biologisch abbaubare Polyurethane, die sich besonders zur Anwendung in der Chirurgie eignen, sind aus der DE-OS 41 11 914 bekannt. Die Röntgenopazität wird durch kovalent gebundene Röntgenkontrastmittel, wie beispielsweise Glycerinmonoestern von Triiodbenzoesäurederivaten, erzielt.

Radikalisch polymerisierbare iodhaltige Methacrylatderivate wurden auch von Brown et al. beschrieben (E. Brown, M. Couturier, J. Touet; *Makromol. Chem*., *Rapid Commun* **6** (1985) 503-7). Die beschriebene Homopolymerisation des Natriumsalzes des 3-Amino-2,4,6-triiodbenzoesäureacrylamids in wäßriger Lösung führt jedoch lediglich zu einem Polymer mit einem mittleren Polymerisationsgrad von etwa 5. Durch die Copolymerisation mit N-[1,1-Bis(hydroxymethyl)-2-hydroxyethyl]acrylamid oder die Verwendung von 3-(3-Acrylamidopropionamido)-2,4,6-triiodbenzoesäure sind zwar Molmassen bis maximal 23 500 g/mol erreichbar, jedoch schmelzen diese hydrophilen iodhaltigen Acrylamide erst bei Temperaturen über 260° C und sind in den üblichen Dentalmonomeren nicht löslich.

Auch die Homo- oder Copolymerisation der 2-Hydroxyethylmethacrylat-Ester substituierter Isophthalsäure, wie beispielsweise 5-Acetamido-2,4,6-triiod-N-methylisophthalamsäure, oder 2,4,6-Triiodphenylmethacrylat mit 2-Hydroxyethylmethacrylat (HEMA) oder Methylmethacrylat (MMA) führt in Gegenwart von Azobisisobutyronitril (AIBN) oder Dibenzylperoxid (DBPO) selbst nach 40 Stunden nur zu oligomeren Produkten (A. Jaykrishnan, B. C. Thanoo; *J*. *Appl. Polym*. *Sci.* **44** (1992) 743-8). Die 4-(1,3,6-Triiod-9-carbazoyl)- und 4-(1,3,6,8-Tetraiod-9-carbazoyl)-1-butylmethacrylate zeichnen sich ebenfalls nur durch eine geringe Polymerisationsneigung aus (R. A. Minns, R. A. Gaudiana; *J*. *Macromol*. *Sci*.- *Pure Appl*. *Chem* **A29** (1992) 19-30) und sind daher auch nicht zum Aufbau einer röntgenopaken Polymermatrix geeignet.
Aufgabe der Erfindung ist deshalb die Schaffung monomerer röntgenopaker Acryl- und Methacrylsäurederivate, die sich radikalisch oder anionisch gut polymerisieren lassen.

Weitere Aufgabe der Erfindung ist die Schaffung röntgenopaker Dentalmaterialien, in denen die röntgenopake Komponente kovalent in das polymere Matrixmaterial eingebunden und damit nicht auswaschbar ist und welche die oben aufgeführten Nachteile bekannter röntgenopaker Füllmaterialien nicht zeigen. Insbesondere sollen die Materialien auch bei geringem oder fehlendem Füllstoffanteil eine ausreichende Röntgenopazität zeigen und hinsichtlich ihrer physikalischen Eigenschaften mit den nichtröntgenopaken Materialien vergleichbar sein. Ferner sollen sie in üblichen Dentalmonomeren gut löslich sein.

Es wurde überraschenderweise gefunden, daß sich die in den Ansprüchen definierten iodsubstituierten Benzoesäureester und -amide sehr gut homo- und copolymerisieren lassen. Die daraus hergestellten Polymere sind in den üblichen Dentalmonomeren gut löslich und darüber hinaus zeigen sowohl die Monomeren als auch die daraus hergestellten Polymere eine hohe UV-Stabilität. Besonders überraschend ist jedoch, daß diese lodderivate eine hervorragende Stabilität aufweisen und zur Erzielung der gewünschten Röntgenopazität allein, d.h. ohne Bromderivate, eingesetzt werden können.

Bevorzugte Derivate sind die Methacrylsäureester (R¹ = CH₃) der 2-Hydroxyethylester (R² = -CH₂-CH₂-) der 3,5-(Diacetylamino)-2,4,6-triiodbenzoesäure (R³, R⁵ und R⁷ = J, R⁴ und R⁶ = NH(COCH₃). Besonders bevorzugte Verbindungen sind 1-(2,3,5-Triiodbenzoyloxy)- und 1-(3,5-Diacetylamino-2,4,6-triiodbenzoyloxy)-2,3-dimethacryloyloxypropan (R¹ = -CH₃; R² = -CH₂CH(-)CH₂-; R³, R⁴, R⁶ = I und R⁵, R⁷ = H bzw. R³, R⁵, R⁷ = I, R⁴, R⁶ = -NH(COCH₃) und n = 2).

Die erfindungsgemäßen röntgenopaken Monomere lassen sich aus triiod- und gegebenenfalls weiter substituierter Benzoesäure durch aus der organischen Chemie bekannte Reaktionen, wie Veresterung oder Veretherung mit Hydroxy- bzw. Halogenalkylacrylaten und -methacrylaten herstellen.

1-(3,5-Diacetylamino-2,4,6-triiodbenzoyloxy)-2,3-dimethacryloyloxypropan (R¹ = -CH₃; R² = -CH₂CH(-)CH₂; R³, R⁵, R⁷ = I, R⁴, R⁶ = -NH(COCH₃), n = 2) kann beispielsweise durch Reaktion des Natriumsalzes der 3,5-Diacetylamino-2,4,6-Triiodbenzoesäure mit 3-Chlorpropan-1,2-diol und nachfolgende Acylierung mit Methacrylsäureanhydrid erhalten werden.

Die Amide lassen sich aus dem Säurechlorid der triiod- und gegebenenfalls weiter substituierten Benzoesäure durch Umsetzung mit polymerisationsfähigen Amiden, wie z. B. Acryl- oder Methacrylamid, nach den in der organischen Chemie bekannten Methoden herstellen.

Weiterer Gegenstand der Erfindung sind Polymere, die aus den erfindungsgemäßen Monomeren allein oder unter Zugabe anderer röntgenopaker und/oder nicht-röntgenopaker Monomere durch radikalische oder anionische Polymerisation erhalten werden können. Als weitere Monomerkomponenten eignen sich besonders mono- oder polyfunktionelle Methacrylate. Bevorzugte Comonomere sind Methylmethacrylat, Triethylenglykoldimethacrylat, Hexandioldimethacrylat, Dodecandioldimethacrylat, Bisphenol-A-dimethacrylat, Bisphenol-A-glycidylmethacrylat, Trimethylolpropantrimethacrylat und Hydroxyethylmethacrylat sowie Urethandimethacrylate, d.h. Umsetzungsprodukte aus Isocyanaten, insbesondere Di- und/oder Triisocyanaten mit hydroxylgruppenhaltigen Methacrylaten. Besonders bevorzugt sind Bisphenol-A-glycidylmethacrylat und das Urethandimethacrylat aus Hydroxyethylmethacrylat und 2,2,4-Trimethylhexamethylendiisocyanat-1,6. Die erfindungsgemäßen Polymere können zu Splitter- oder Perlpolymerisaten verarbeitet werden, die als Füllstoffe für Dentalmaterialien eingesetzt werden können. Typischerweise beträgt der Anteil der erfindungsgemäßen Monomere 10 bis 100 Gew.-%, vorzugsweise 20 bis 60 Gew.-%. an der Gesamtmasse der erfindungsgemäßen Polymere.

Weiterer Gegenstand der Erfindung sind röntgenopake Dentalmaterialien, die unter Verwendung der erfindungsgemäßen Monomeren und/oder Polymeren erhalten werden. Diese eignen sich als Füllungskomposites, Befestigungszemente, Haftvermittler (Bondings) sowie zur Herstellung künstlicher Zähne, Inlays, Implantate, Kronen, Brücken und Fertigteilen, vorzugsweise als Zahnfüllmaterial, Befestigungszement oder Bonding.

Durch die Anwendung der erfindungsgemäßen Dentalmaterialien soll der Zahnarzt beim Anfertigen von Röntgenbildern in die Lage versetzt werden, aufgrund der Röntgenopazität die Zement- oder Bonding-Schicht vom eventuell vorhandenen Randspalt zu unterscheiden.

Die erfindungsgemäßen Zahnfüllmaterialien, Befestigungszemente oder Bondings enthalten die in den Ansprüchen definierten erfindungsgemäßen Bezoesäurederivate bevorzugt in monomerer Form. Ihr Anteil an der Gesamtmasse des Dentalmaterials hängt von der gewünschten Röntgenopazität ab und liegt im Bereich von 5 bis 100 Gew.-%, bevorzugt im Bereich von 20 bis 90 Gew.-% und besonders bevorzugt im Bereich von 40 bis 70 Gew.-%.

Die erfindungsgemäßen Dentalmaterialien zur Herstellung von Fertigteilen wie künstlichen Zähnen, Inlays, Kronen, Brücken, Schalen (veneers) und Implantaten etc. enthalten die in den Ansprüchen definierten erfindungsgemäßen Benzoesäurederivate bevorzugt in polymerisierter Form. Ihr Anteil an der Gesamtmasse des Dentalmaterials ist unter anderem abhängig vom Vorhandensein eventuell vorhandener röntgenopaker Füllstoffe und liegt im Bereich von 5 bis 90 Gew.-%, bevorzugt im Bereich von 10 bis 70 Gew.-% und besonders bevorzugt im Bereich von 20 bis 50 Gew.-%.

Als weitere Komponenten können die erfindungsgemäßen Dentalmaterialien Monomere, die zur Copolymerisation mit den erfindungsgemäßen Monomeren geeignet sind, enthalten. Bevorzugt sind mono- oder polyfunktionelle Methacrylate sowie die oben zur Herstellung der erfindungsgemäßen Polymere als bevorzugt aufgeführten Verbindungen.

Darüber hinaus können die erfindungsgemäßen Dentalmaterialien auch Polymerisate der erfindungsgemäßen Monomere in Dimethacrylat oder einem anderen Monomeren gelöst, suspendiert oder gequollen, enthalten.

Das Dentalmaterial kann je Art des verwendeten Initiators heiß, kalt oder durch Photopolymerisation ausgehärtet werden.

Als Initiatoren für die Heißpolymerisation eigenen sich die bekannten Peroxide wie Dibenzoylperoxid, Dilaurylperoxid, tert.-Butylperoctoat oder tert.-Butylperbenzoat sowie Azobistsobutyroethylester. Benzpinakol und 2.2'-Dimethylbenzpinakol. Bevorzugt sind Dibenzoyl- und Dilaurylperoxid.

Als Initiatoren für die Kaltpolymerisation werden Radikale liefernde Systeme, zum Beispiel Benzoyl- bzw. Laurylperoxid zusammen mit Aminen, wie N,N-Dimethyl-sym.-xylidin oder N,N-Dimethyl-p-toluidin eingesetzt. Darüber hinaus sind Verbindungen geeignet, die wie Trimethylsilylketenacetale in Gegenwart von nukleophilen Katalysatoren oder Lewis-Säuren die Gruppen-Transfer-Polymerisation von Methacrylaten initiieren. Bevorzugte Trimethylsilylketenacetale sind (1-Methoxy-2-methyl-1-propenyloxy)trimethylsilan und Bis(1-methoxy-2-methyl-1-propenoxy)methylsilan. Bevorzugte nukleophile Katalysatoren sind Tetrabutylammoniumcyanid sowie Tris(dimethylamino)sulfoniumbifluorid. Als Lewis-Säuren werden Zinkbromid und Diisobutylaluminiumchlorid bevorzugt.

Als Initiatoren für die Photopolymerisation können beispielsweise Benzophenon und Benzoin sowie deren Derivate verwendet werden. Darüber hinaus stellen auch α-Diketone geeignete Photoinitiatoren dar. Bevorzugt sind 9,10-Phenanthrenchinon, Diacetyl- oder 4 4'-Dichlorbenzil. Besonders bevorzugt ist Campherchinon. Die α-Diketone werden bevorzugt in Kombination mit Aminen als Reduktionsmittel eingesetzt Bevorzugte Amine sind Cyanoethylmethylanilin, Dimethylaminoethylmethacrylat. Triethanolamin und N,N-Dimethyl-sym.-xylidin. Das Verhältnis von Initiator zu Amin beträgt im allgemeinen 1:1. Das am meisten bevorzugte Photoinitiatorsystem enthält 0.3 Gew,-% Campherchinon und 0.5 Gew.-% Cyanoethylmethylanilin.

Bei der Verwendung von Photoinitiatoren erhalten die erfindungsgemäßen Dentalmassen neben den erfindungsgemäßen Monomeren vorzugsweise Urethandimethacrylat und/oder Bisphenol-A-glycidylmethacrylat in einer Menge bezogen auf die Gesamtmasse des Dentalmaterials von 0 bis 95 Gew.-%, bevorzugt von 30 bis 60 Gew.-%, als weitere Vernetzerkomponente sowie Triethylenglykoldimethacrylat in einer Menge von 0 bis 30 Gew.-% als verdünnendes Vernetzermonomer.

Zur Verwendung in Zahnfüllmaterialien, Befestigungszementen und Bondings sind Kalt- und Photoinitiatoren bevorzugt, wobei Photoinitiatoren besonders bevorzugt sind. Die radikalischen Initiatoren werden üblicherweise in einer Menge von 0,1 bis 5,0 Gew.-%, vorzugsweise von 0,3 bis 2,0 % bezogen auf die Gesamtmasse des Dentalmaterials eingesetzt.

Die erfindungsgemäßen Dentalmaterialien können darüber hinaus noch andere in der Dentalchemie übliche Zuschlagstoffe, wie anorganische und organische, röntgenopake oder nicht-röntgenopake Zuschlagstoffe, wie Füllstoffe, Pigmentierungsmittel und Stabilisatoren enthalten (vgl. J. Viohl, K. Dermann, D. Quast, S. Venz, *Die Chemie der zahnärztlichen Füllkunststoffe*, Hanser-Verlag, München-Wien, 1986, S. 7 ff.).

Als anorganische, nicht-röntgenopake Füllstoffe eignen sich beispielsweise amorphe Kieselsäuren. Bevorzugt ist pyrogene oder gefällte Kieselsäure mit einer BET-Oberfläche von 30 bis 300 m²/g. Als röntgenopake anorganische Füllstoffe eignen sich röntgenopake Gläser, Bariumsulfat oder Ytterbiumfluorid. Die anorganischen Bestandteile sind vorzugsweise in der üblichen Weise, zum Beispiel mit 3-Methacryloyloxypropyltrimethoxysilan, silanisiert.

Als organische Füllmaterialien können dem Dentalmaterial feinteilige Splitter- oder Perlpolymerisate zugesetzt werden. Diese Homo- bzw. Copolymere der üblichen mono- oder polyfunktionellen Methacrylate können ihrerseits mit den beschriebenen röntgenopaken oder nichtröntgenopaken anorganischen Füllstoffen gefüllt sein. Vorzugsweise werden sie unter Verwendung der erfindungsgemäßen Monomeren und/oder Polymeren hergestellt.

Die Zahnfüllungsmaterialien und Fertigteile wie z.B. Inlays werden nach den bekannten Verfahren unter Verwendung der erfindungsgemäßen Monomeren hergestellt (vgl. u.a. EP 0 189 540 oder K. Körber, K. Ludwig, *Zahnärztliche Werkstoffkunde und Technologie,* Thieme-Verlag, Stuttgart-New York 1982, S. 53 ff.).

Die Erfindung bezieht sich nicht nur auf den röntgenopaken Dentalwerkstoff, sondern auch auf daraus hergestellte Fertigtelle, wie beispielsweise künstliche Zähne, Schalen (veneers), Implantaten, Kronen, Brücken, Inlays, usw. Die Erfindung wird anhand der nachfolgenden Beispiele näher erläutert.

### Ausführungsbeispiele

### Beispiel 1

### Synthese von 2-Methacryloyloxyethyl-2.3.5-triiodbenzoat (1): (Vergleichsbeispiel)

Eine Lösung von 2.96 g (22 mmol) über wasserfreiem Natriumsulfat getrocknetem 2-Hydroxyethylmethacrylat, 2.29 ml (20 mmol) wasserfreiem Triethylamin und 0.5 g 4-Dimethylaminopyridin (DMAP) in 100 ml absolutem Tetrahydrofuran (THF) wird bei 0 bis 5° C tropfenweise mit einer Lösung von 10,69 g (20 mmol) 2,3,5-Triiodbenzoylchlorid, das durch Umsetzung von 2.3.5-Triiodbenzoesäure mit Thionylchlorid zugänglich ist (analog Organikum, 12. Aufl., Deutscher Verlag der Wissenschaften, Berlin 1973, S.469), in 100 ml THF versetzt. Die Reaktionsmischung wird 40 Stunden bei Raumtemperatur aufbewahrt und anschließend im Vakuum eingeengt. Nach Zugabe von Methylenchlorid wird die Mischung nacheinander mit verdünnter Salzsäure, 10 %-iger NaHCO₃-Lösung und Wasser gewaschen und dann über wasserfreiem Natriumsulfat getrocknet. Das durch Einengen der Lösung erhaltene Rohprodukt wird zweimal aus Ethanol in Gegenwart von Aktivkohle umkristallisiert; 7,5 g (59 %) farblose Kristalle mit einem Schmelzpunkt von 99° C, der mittels Differential Scanning Calorimetry (DSC) bestimmt wurde. 1 g der Substanz wird zu einer Tablette gepreßt (Durchmesser ca. 12 mm Schichtdicke 2 mm) und die Tablette 30 Minuten mit dem ganzen Wellenlängenbereich eines Suntest-Gerätes CPS (Heraeus) mit einer Bestrahlungsstärke von ca. 765 W/m² bestrahlt. Dabei tritt keine Verfärbung der Tablette auf.

| | | | | |
|---|---|---|---|---|
| C₁₃H₁₁O₄J₃ (611.94 g/mol) | Gef | C 25.51 | H 1.17 | J 62.14 |
| | Ber | C 25.52 | H 1.81 | J 62.21 |

¹H-NMR (300 MHz, CDCl₃, in ppm); 1,97 (s, 3H, =C(CH₃)-); 4,49 und 4,58 (2t, 4H, -CH₂-CH₂-); 5,62 und 6,17 (2s, 2H, =CH₂); 7.35 und 8.31 (2s. 2H. aromat. H).
IR (Film, in cm⁻¹); 1723 (C=O), 1639 (C=C).

### Beispiel 2

### Synthese von 2,3-Dimethacryloyloxypropyl-1-(2,3,5-triiodbenzoyloxy)propan (2):

Analog Beispiel 1 werden 5.04 g (21,9 mmol) Glyceroldimethacrylat mit 11.40 g (21.9 mmol) 2,3,5-Triiodbenzoylchlorid in Gegenwart von Triethylamin und Dimethylaminopyridin in THF umgesetzt. Die Aufarbeitung des Reaktionsansatzes erfolgt wie in Beispiel 1 beschrieben. Es werden 11,9 g (83.6 %) einer viskosen Flüssigkeit erhalten. 1 g der Substanz wird analog Beispiel 1 bestrahlt (Schichtdicke ca. 2 mm), wobei keine Verfärbung auftritt.

| | | | | |
|---|---|---|---|---|
| C₁₈H₁₇O₆J₃ (710,04 g/mol). | Gef. | C 30,85 | H 2,76 | J 51,10 |
| | Ber. | C 30,45 | H 2,41 | J 53,62 |

¹H-NMR (90 Mhz, CDCl₃, in ppm): 1.97 (s, 3H, =C(CH₃)-); 3,73 (m, 1H, =CH-O); 4,45 (t, 4H, -CH₂-O); 5,62 und 6,14 (2s, 2H, =CH₂); 7.67 und 8,30 (2s, 2H. aromat. H).
IR (Film, in cm⁻¹): 1724 (C=O), 1637 (C=C).

### Beispiel 3

### Synthese von 1-(3,5-Diacetylamino-2,4,6-triiodbenzoyloxy)-2,3-dimethacryloyloxypopan (3):

20 g (29 mmol) 3.5-Diacetylamino-2,4,6-triiodbenzoesäure-2,3-dihydroxypropylester, der aus dem Natriumsalz der 3,5-Diacetylamino-2,4,6-triiodbenzoesäure durch Umsetzung mit 3-Chlorpropan-1.2-diol bei 105° C zugänglich ist (analog Organikum, 12. Auflage, Deutscher Verlag der Wissenschaften, Berlin 1973, S. 227 f.), werden in 80 ml absolutem Pyridin gelöst und auf -10° C abgekühlt. Zu dieser Lösung tropft man 20 g (130 mmol) käufliches Methacrylsäureanhydrid (Fluka) und rührt anschließend bei Raumtemperatur über Nacht. Die resultierende klare Reaktionsmischung wird mit 1 l verdünnter Salzsäure versetzt und der sich bildende weiße Niederschlag abfiltriert, mit Wasser neutral gewaschen und anschließend aus Methanol/Wasser umgefällt. Nach Trocknen im Feinvakuum (ca. 1 mbar, bei Raumtemperatur) bis zur Gewichtskonstanz werden 16,1 g (67,1 %) farbloses, amorph erscheinendes Pulver vom DSC-Schmelzpunkt 207° C erhalten. 1 g der Substanz wird wie in Beispiel 1 beschrieben zu einer Tablette gepreßt und bestrahlt, es tritt keine Verfärbung auf.

| | | | | | |
|---|---|---|---|---|---|
| C₂₂H₂₃N₂O₈J₃ (824,1 g/mol) | Gef. | C 32,23 | H 2,91 | N 3,39 | J 45,92 |
| | Ber. | C 32,06 | H 2,81 | N 3,40 | J 46,19 |

¹H-NMR (300 MHz, [D₆]DMSO, in ppm): 1,86 und 1,87 (2s, 6H, =C(CH₃)-; 2,03 (s, 6H, -CO-CH₃); 4,34-4,55 (m, 4H, -CH₂-); 5,45 (m, 1H, =CH-); 5,70 und 6,05 (2s, 4H, =CH₂); 9,99 und 10,08 (2s, 2H, -NH-CO-).

### Beispiel 4

### Lösungspolymerisation der Monomere 1, 2 und 3:

Die Monomere werden in den in Tabelle 1 angegebenen Konzentrationen in Schlenkgefäßen in Dimethylformamid (DMF) gelöst. Nach Zugabe von Azobisisobutyronitril (AIBN. 40 mmol/1) werden die Gefäße verschlossen und durch einen dreimaligen Einfrier- und Auftauzyklus unter Argon oder Stickstoff von Sauerstoff befreit und anschließend im Thermostaten auf 60° C erwärmt. Die Polymerisation wird nach den in Tabelle 1 gezeigten Zeiten durch Abkühlen der Polymerisationslösung in einer Trockeneis-Acetonmischung abgebrochen und das Polymerisat durch Eingießen in einen etwa 10-fachen Überschuß Methanol ausgefällt. Der Niederschlag wird abfiltriert und im Feinvakuum (ca. 1 mbar. Raumtemperatur) bis zur Gewichtskonstanz getrocknet. Im Fall des Monomethacrylats 1 bilden sich lösliche Homopolymere, die vor der Molmassebestimmung aus THF/Methanol umgefällt werden. Tabelle 1 zeigt die erzielten Umsätze und Molmassen.

**Tabelle 1**

| **Lösungspolymerisation der Monomere 1, 2 und 3** | | | | | |
|---|---|---|---|---|---|
| Monomer | Konz. des Monomers (mol/l) | Zeit (min) | Umsatz (%) | Molmasse^{a)} Mₙ.10⁻³ (g/mol) | Polymerisationsgrad Pₙ=Mₙ/M₀^{d)} |
| 1 | 1,00 | 15 | 42,1 | 146,0 | 238,6 |
| 1 | 1,00 | 30 | 68,1 | 112,3 | 183,5 |
| 1 | 1,00 | 60 | 87,5 | 86,00 | 140,5 |
| 1 | 0,50 | 60 | 73.5 | 58,9 | 96,3 |
| 2 | 0,50 | 15 | 68,7 | -^{b)} | - |
| 2 | 0,50 | 30 | 86,0 | - | - |
| 3 | 0,20 | 60 | 73,2 | - | - |
| 3 | 0,50 | 60 | 89,9 | - | - |
| 3 | 1,00 | 60 | 92,7 | -^{c)} | - |

| | | | | | |
|---|---|---|---|---|---|
| ^{a)} Bestimmt mittels Gelpermeationschromatograhpie (GPC) mit PMMA-Standards | | | | | |
| ^{b)} Gelzeit: 3 Minuten | | | | | |
| ^{c)} Gelzeit: 12 Minuten | | | | | |
| ^{d)} M₀ = Molmasse des Monomers: M₀ = 611.94 g/mol für Monomer 1: | | | | | |

### Beispiel 5

### Herstellung von röntgenopaken Dentalmaterialien durch Copolymerisation der Monomere 1, 2 oder 3 mit herkömmlichen Dentalmonomeren:

Zur Bestimmung der Röntgenopazität (RO) gemäß ISO-Norm 4049 ("Dentistry - Resin-based filling materials", Seite 1) werden die erfindungsgemäßen Monomere **1** bis **3** in den nachfolgend angegebenen Verhältnissen mit herkömmlichen Monomeren und einem Photoinitiator gemischt und die Mischungen zu Stäbchen geformt (2 mm x 4 mm x 25 mm), die durch zweimaliges Bestrahlen für je 3 Minuten in einem Lichtpolymerisationsgerät (Spectramat, Ivoclar AG) polymerisiert werden (Wellenlänge: 400 - 500 nm; Lichtintensität: ca. 200 mW/cm²). Als Photoinitiator wird eine Mischung aus Campherchinon und Cyanoethylmethylanilin in einer Menge von 0,3 bis 0,5 Gewichts% eingesetzt. Die Bestimmung der Röntgenopazität erfolgte durch Vergleich mit Aluminiumplatten gleicher Schichtdicke.

| | | |
|---|---|---|
| a) | Monomer **3** | 25 Gew.-% |
| | 2-Hydroxyethylmethacrylat (HEMA) | 74,2 % |
| | Initiatormischung | 0,8 % |
| | Röntgenopazität | 25 % Aluminium |
| | | |
| b) | Monomer **1** | 23,0 % |
| | Triethylenglykoldimethacrylat (TEGDMA) | 65,7 % |
| | Dimethylformamid | 10,5 % |
| | Initiatormischung | 0,8 % |
| | Röntgenopazität | 100 % Aluminium |
| | | |
| c) | Monomer **2** | 49,8 % |
| | TEGDMA | 49,7% |
| | initiatormischung | 0,5 % |
| | Röntgenopazität | 200 % Aluminium |

Gleiche Werte für die Röntgenopazität (200 % Aluminium) werden erzielt, wenn in Beispiel c) TEGDMA durch gleiche Anteile Bisphenol-A-Glycidyl-methacrylat. Dodecandioldimethacrylat oder das Addukt aus Hydroxyethylmethacrylat und 2,2,4-Trimethylhexamethylendiisocyanat ersetzt wird.

## Patentansprüche (Patentansprüche für folgende(n) Vertragsstaat(en): CH, DE, FR, GB, IT, LI, SE)

1. Röntgenopake Ester oder Amide iodsubstituierter Benzoesäure gemäß der Formel 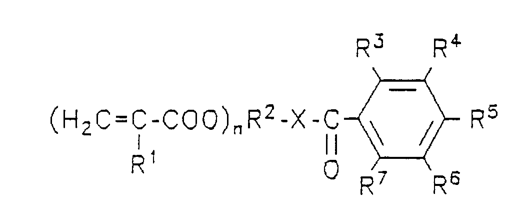 in welcher
R¹ = Wasserstoff oder C₁- bis C₃-Alkyl;
R² = geradkettiges oder verzweigtes C₁- bis C₆-Alkylen, -Oxyalkylen oder -Arylen;
X = O oder NH;
R³-R⁷ = mindestens 3 Iodsubstituenten, und die übrigen Reste Wasserstoff, C₁- bis C₆-Alkyl, C₁- bis C₆-Alkoxy, -Cl, -Br, -OH, -NH₂, -N(C₁- bis C₆-Alkyl)₂ oder -NH-CO-(C₁- bis C₆-Alkyl) sind; und
n = 1, 2 oder 3 ist,
wobei x nicht 0 sein kann wenn n gleich 1, R² geradkettiges oder verzweigtes C₁- bis C₄-Alkylen oder C₁-C₆-oxyalkylen und R¹ Wasserstoff oder Methyl ist und R³-R⁷ Iod, Brom oder Wasserstoff sind.

2. Benzoesäurederivate nach Anspruch 1, **dadurch gekennzeichnet,** daß
R¹ = H oder CH₃;
R² = geradkettiges oder verzweigtes C₂- bis C₄-Alkylen;
X₃ = O;
R³, R⁴ und R⁶ = I und
R⁵ und R⁷ = H, oder
R³, R⁵ und R⁷ = I und
R⁴ und R⁶ = NH(COCH₃) sind und
n = 1 oder 2 ist.

3. Benzoesäurederivate nach Anspruch 1, **dadurch gekennzeichnet,** daß
R¹ = CH₃;
R² = -CH₂CH₂- oder -CH₂CH(-)CH₂-;
X = O;
R³, R⁴ und R⁶ = I und
R⁵ und R⁷ = H, oder
R³, R⁵ und R⁷ = I und
R⁴ und R⁶ = NH(COCH₃) sind und
n = 1 oder 2 ist.

4. Röntgenopake Acrylat- und/oder Methacrylatpolymere, **dadurch gekennzeichnet,** daß sie unter Verwendung mindestens einer Verbindung gemäß einem der Ansprüche 1 bis 3 als Monomerkomponente hergestellt sind.

5. Röntgenopake Acrylat- und/oder Methacrylatpolymere nach Anpruch 4, **dadurch gekennzeichnet,** daß der Anteil der Monomerkomponente(n) gemäß einem der Anprüche 1 bis 3 an der Gesamtmasse des Polymeren 20 bis 60 Gew-% beträgt.

6. Röntgenopake Acrylat- und/oder Methacrylatpolymere nach einem der Ansprüche 4 oder 5, **dadurch gekennzeichnet,** daß sie unter Verwendung eines oder mehrerer mono- und/oder polyfunktioneller Methacrylate als Comonomere hergestellt sind.

7. Röntgenopake Acrylat- und/oder Methacrylatpolymere, **dadurch gekennzeichnet,** daß sie unter Verwendung von mindestens einem Ester oder Amid iodsubstituierter Benzoesäure gemäß der Formel 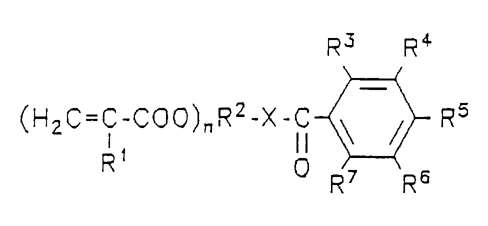 in welcher
R¹ = Wasserstoff oder C₁- bis C₃-Alkyl;
R² = geradkettiges oder verzweigtes C₁- bis C₆-Alkylen, -Oxyalkylen oder -Arylen;
X = O oder NH;
R³-R⁷ = mindestens 3 Iodsubstituenten, und die übrigen Reste Wasserstoff, C₁- bis C₆-Alkyl, C₁- bis C₆-Alkoxy, -Cl, -Br, -OH, -NH₂, -N(C₁- bis C₆-Alkyl)₂ oder -NH-CO-(C₁- bis C₆-Alkyl) sind; und
n = 1, 2 oder 3 ist,
als Monomerkomponente und Bisphenol-A-glycidylmethacrylat und/oder dem Urethandimethacrylat aus Hydroxyethylmethacrylat und 2,2,4-Trimethylhexamethylendiisocyanat-1,6 als Comonomer hergestellt sind.

8. Röntgenopakes Dentalmaterial, **dadurch gekennzeichnet,** daß es mindestens ein röntgenopakes Monomer gemäß einem der Ansprüche 1 bis 3 und/oder röntgenopakes Polymer nach einem der Ansprüche 4 bis 7 enthält.

9. Röntgenopakes Dentalmaterial, **dadurch gekennzeichnet,** daß es mindestens einen Ester oder ein Amid iodsubstituierter Benzoesäure gemäß der Formel 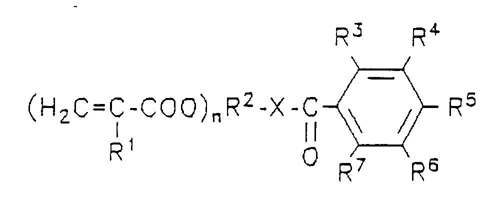 in welcher
R¹ = Wasserstoff oder C₁- bis C₃-Alkyl;
R² = geradkettiges oder verzweigtes C₁- bis C₆-Alkylen, -Oxyalkylen oder -Arylen;
X = O oder NH;
R³-R⁷ = mindestens 3 Iodsubstituenten, und die übrigen Reste Wasserstoff, C₁- bis C₆-Alkyl, C₁- bis C₆-Alkoxy, -C₁, -Br, -OH, -NH₂, -N(C₁- bis C₆-Alkyl)₂ oder -NH-CO-(C₁- bis C₆-Alkyl) sind; und
n = 1, 2 oder 3 ist,
und/oder röntgenopakes Polymer nach einem der Ansprüche 4 bis 7 und einen Initiator für die Photopolymerisation enthält.

10. Röntgenopakes Dentalmaterial nach Anspruch 8, **dadurch gekennzeichnet,** daß es 5 bis 100 Gew.-% eines Monomers oder Mischung von Monomeren nach einem der Ansprüche 1 bis 3 enthält.

11. Dentalmaterial gemäß einem der Ansprüche 8 bis 10, **dadurch gekennzeichnet,** daß es
als Initiator für die Heißpolymerisation Dibenzoylperoxid und/oder Dilaurylperoxid enthält oder daß es
als Initiator für die Kaltpolymerisation Benzoylperoxid, Laurylperoxid und N,N-Dimethyl-sym.-xylidin oder N,N-Dimethyl-p-toluidin; oder (1-Methoxy-2-methyl-1-propenyloxy)trimethylsilan oder Bis(1-methoxy-2-methyl-1-propenoxy)methylsilan und Tetrabutylammoniumcyanid oder Zinnbromid oder Tetrabutylammoniumcyanid oder Tris(dimethylamino)sulfoniumbifluorid enthält; oder daß es
als Initiator für die Photopolymerisation ein Benzophenon, Benzoin und/oder ein α-Diketon enthält.

12. Dentalmaterial gemäß Anspruch 11, **dadurch gekennzeichnet,** daß es einen Photoinitiator, Triethylenglykoldimethacrylat und bezogen auf die Gesamtmasse des Dentalmaterials bis zu 95 Gew.-% Urethandimethacrylat und/oder Bisphenol-A-glycidylmethacrylat enthält.

13. Dentalmaterial gemäß Anspruch 12, **dadurch gekennzeichnet,** daß es 30 bis 60 Gew.-% Urethandimethacrylat und/oder Bisphenol-A-glycidylmethacrylat enthält.

14. Verwendung von Dentalmaterialien nach einem der Ansprüche 8 bis 13 als Zahnfüllmaterial, Befestigungzement und/oder Bonding.

15. Verwendung von Benzoesäurederivaten nach einem der Ansprüche 1 bis 3 und/oder von Acrylat- und/oder Methacrylatpolymeren nach einem der Ansprüche 4 bis 7 zur Herstellung von röntgenopaken Dentalmaterialien, wie Füllungskompositen, Befestigungszementen, Haftvermittlern (Bondings), künstlichen Zähnen, Inlays, Implantaten, Kronen, Brücken oder Fertigteilen.

16. Verwendung von Benzoesäurederivaten nach einem der Ansprüche 1 bis 3 und/oder von Acrylat- und/oder Methacrylatpolymeren nach einem der Ansprüche 4 bis 7 zur Herstellung von röntgenopaken Zahnfüllmaterialien.

17. Künstliche Zähne, Schalen (veneers), Implantate, Kronen, Brücken oder Inlays, **dadurch gekennzeichnet,** daß sie unter Venwendung von mindestens einem Monomeren gemäß einem der Ansprüche 1 bis 3 und/oder Polymeren nach einem der Ansprüche 4 bis 7 und/oder Dentalmaterialien nach einem der Ansprüche 8 bis 13 hergestellt wurden.

## Patentansprüche (Patentansprüche für folgende(n) Vertragsstaat(en): AT)

1. Röntgenopake Ester oder Amide iodsubstituierter Benzoesäure gemäß der Formel 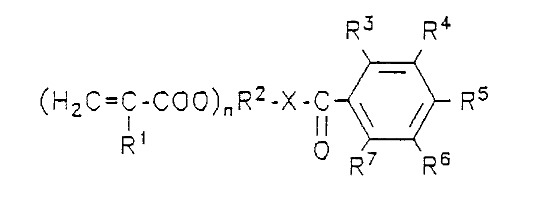 in welcher
R¹ = Wasserstoff oder C₁- bis C₃-Alkyl;
R² = geradkettiges oder verzweigtes C₁- bis C₆-Alkylen, -Oxyalkylen oder -Arylen;
X = O oder NH;
R³-R⁷ = mindestens 3 Iodsubstituenten, und die übrigen Reste Wasserstoff, C₁- bis C₆-Alkyl, C₁- bis C₆-Alkoxy, -Cl, -Br, -OH, -NH₂, -N(C₁- bis C₆-Alkyl)₂ oder -NH-CO-(C₁- bis C₆-Alkyl) sind; und
n = 1, 2 oder 3 ist,
wobei X nicht O sein kann, wenn n gleich I, R² Ethylen oder 1,2-Propylen oder geradkettiges oder verzweigtes C₁- bis C₆-Oxyalkylen und R¹ Wasserstoff oder Methyl ist und R³-R⁷ lod oder Wasserstoff sind.

2. Benzoesäurederivate nach Anspruch 1, **dadurch gekennzeichnet,** daß
R¹ = H oder CH₃;
R² = geradkettiges oder verzweigtes C₂- bis C₄-Alkylen;
X₃ = O;
R³, R⁴ und R⁶ = I und
R⁵ und R⁷ = H, oder
R³, R⁵ und R⁷ = I und
R⁴ und R⁶ = NH(COCH₃) sind und
n = 1 oder 2 ist.

3. Benzoesäurederivate nach Anspruch 1, **dadurch gekennzeichnet,** daß
R¹ = CH₃;
R² = -CH₂CH₂- oder -CH₂CH(-)CH₂-;
X₃ = O;
R³, R⁴ und R⁶ = I und
R⁵ und R⁷ = H, oder
R³, R⁵ und R⁷ = I und
R⁴ und R⁶ = NH(COCH₃) sind und
n = 1 oder 2 ist.

4. Röntgenopake Acrylat- und/oder Methacrylatpolymere, **dadurch gekennzeichnet,** daß sie unter Verwendung mindestens einer Verbindung gemäß einem der Ansprüche 1 bis 3 als Monomerkomponente hergestellt sind.

5. Röntgenopake Acrylat- und/oder Methacrylatpolymere nach Anpruch 4, **dadurch gekennzeichnet,** daß der Anteil der Monomerkomponente(n) gemäß einem der Anprüche 1 bis 3 an der Gesamtmasse des Polymeren 20 bis 60 Gew-% beträgt.

6. Röntgenopake Acrylat- und/oder Methacrylatpolymere nach einem der Ansprüche 4 oder 5, **dadurch gekennzeichnet,** daß sie unter Verwendung eines oder mehrerer mono- und/oder polyfunktioneller Methacrylate als Comonomere hergestellt sind.

7. Röntgenopake Acrylat- und/oder Methacrylatpolymere, **dadurch gekennzeichnet,** daß sie unter Verwendung von mindestens einem Ester oder Amid iodsubstituierter Benzoesäure gemäß der Formel 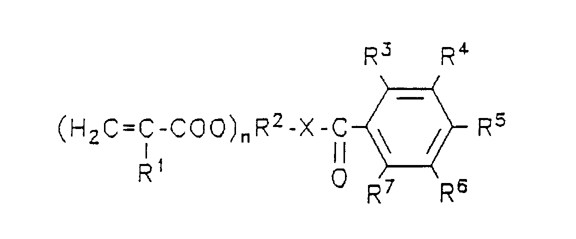 in welcher
R¹ = Wasserstoff oder C₁- bis C₃-Alkyl;
R² = geradkettiges oder verzweigtes C₁- bis C₆-Alkylen, -Oxyalkylen oder -Arylen;
X = O oder NH;
R³-R⁷ = mindestens 3 Iodsubstituenten, und die übrigen Reste Wasserstoff, C₁- bis C₆-Alkyl, C₁- bis C₆-Alkoxy, -Cl, -Br, -OH, -NH₂, -N(C₁- bis C₆-Alkyl)₂ oder -NH-CO-(C₁- bis C₆-Alkyl) sind; und
n = 1, 2 oder 3 ist,
als Monomerkomponente und Bisphenol-A-glycidylmethacrylat und/oder dem Urethandimethacrylat aus Hydroxyethylmethacrylat und 2,2,4-Trimethylhexamethylendiisocyanat-1,6 als Comonomer hergestellt sind.

8. Röntgenopakes Dentalmaterial, **dadurch gekennzeichnet,** daß es mindestens ein röntgenopakes Monomer gemäß einem der Ansprüche 1 bis 3 und/oder röntgenopakes Polymer nach einem der Ansprüche 4 bis 7 enthält.

9. Röntgenopakes Dentalmaterial, **dadurch gekennzeichnet,** daß es mindestens einen Ester oder ein Amid iodsubstituierter Benzoesäure gemäß der Formel 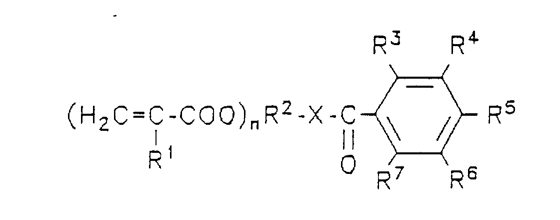 in welcher
R¹ = Wasserstoff oder C₁- bis C₃-Alkyl;
R² = geradkettiges oder verzweigtes C₁- bis C₆-Alkylen, -Oxyalkylen oder -Arylen;
X = O oder NH;
R³-R⁷ = mindestens 3 Iodsubstituenten, und die übrigen Reste Wasserstoff, C₁- bis C₆-Alkyl, C₁- bis C₆-Alkoxy, -Cl, -Br, -OH, -NH₂, -N(C₁- bis C₆-Alkyl)₂ oder -NH-CO-(C₁- bis C₆-Alkyl) sind; und
n = 1, 2 oder 3 ist,
und/oder röntgenopakes Polymer nach einem der Ansprüche 4 bis 7 und einen Initiator für die Photopolymerisation enthält.

10. Röntgenopakes Dentalmaterial nach Anspruch 8, **dadurch gekennzeichnet,** daß es 5 bis 100 Gew.-% eines Monomers oder Mischung von Monomeren nach einem der Ansprüche 1 bis 3 enthält.

11. Dentalmaterial gemäß einem der Ansprüche 8 bis 10, **dadurch gekennzeichnet,** daß es
als Initiator für die Heißpolymerisation Dibenzoylperoxid und/oder Dilaurylperoxid enthält oder daß es
als Initiator für die Kaltpolymerisation Benzoylperoxid, Laurylperoxid und N,N-Dimethyl-sym.-xylidin oder N,N-Dimethyl-p-toluidin; oder (1-Methoxy-2-methyl-1-propenyloxy)trimethylsilan oder Bis(1-methoxy-2-methyl-1-propenoxy)methylsilan und Tetrabutylammoniumcyanid oder Zinnbromid oder Tetrabutylammoniumcyanid oder Tris(dimethylamino)sulfoniumbifluorid enthält; oder daß es
als Initiator für die Photopolymerisation ein Benzophenon, Benzoin und/oder ein α-Diketon enthält.

12. Dentalmaterial gemäß Anspruch 11, **dadurch gekennzeichnet,** daß es einen Photoinitiator, Triethylenglykoldimethacrylat und bezogen auf die Gesamtmasse des Dentalmaterials bis zu 95 Gew.-% Urethandimethacrylat und/oder Bisphenol-A-glycidylmethacrylat enthält.

13. Dentalmaterial gemäß Anspruch 12, **dadurch gekennzeichnet,** daß es 30 bis 60 Gew.-% Urethandimethacrylat und/oder Bisphenol-A-glycidylmethacrylat enthält.

14. Verwendung von Dentalmaterialien nach einem der Ansprüche 8 bis 13 als Zahnfüllmaterial, Befestigungzement und/oder Bonding.

15. Verwendung von Benzoesäurederivaten nach einem der Ansprüche 1 bis 3 und/oder von Acrylat- und/oder Methacrylatpolymeren nach einem der Ansprüche 4 bis 7 zur Herstellung von röntgenopaken Dentalmaterialien, wie Füllungskompositen, Befestigungszementen, Haftvermittlern (Bondings), künstlichen Zähnen, Inlays, Implantaten, Kronen, Brücken oder Fertigteilen.

16. Verwendung von Benzoesäurederivaten nach einem der Ansprüche 1 bis 3 und/oder von Acrylat- und/oder Methacrylatpolymeren nach einem der Ansprüche 4 bis 7 zur Herstellung von röntgenopaken Zahnfüllmaterialien.

17. Künstliche Zähne, Schalen (veneers), Implantate, Kronen, Brücken oder Inlays, **dadurch gekennzeichnet,** daß sie unter Venwendung von mindestens einem Monomeren gemäß einem der Ansprüche 1 bis 3 und/oder Polymeren nach einem der Ansprüche 4 bis 7 und/oder Dentalmaterialien nach einem der Ansprüche 8 bis 13 hergestellt wurden.

## Claims (Claims for the following Contracting State(s): CH, DE, FR, GB, IT, LI, SE)

1. X-ray opaque esters or amides of iodine-substituted benzoic acid of the formula 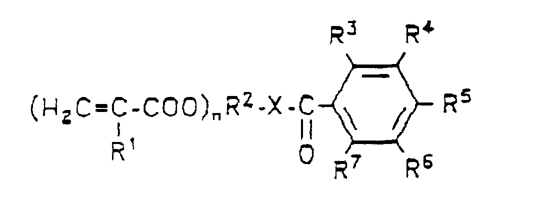 in which
R¹ = hydrogen or C₁- to C₃-alkyl;
R² = straight-chain or branched C₁- to C₆-alkylene, -oxyalkylene or -arylene;
X = O or NH;
R³-R⁷ = at least 3 iodine substituents, and the other radicals are hydrogen, C₁- to C₆-alkyl, C₁- to C₆-alkoxy, -Cl, -Br, -OH, -NH₂, -N(C₁- to C₆-alkyl)₂ or -NH-CO-(C₁- to C₆-alkyl); and
n = 1, 2 or 3,
where X can not be O when n is equal to 1, R² is straight-chain or branched C₁- to C₄-alkylene or C₄-C₆-oxyalkylene and R¹ is hydrogen or methyl and R³-R⁷ are iodine, bromine or hydrogen.

2. Benzoic acid derivatives according to Claim 1, characterized in that
R¹ = H or CH₃;
R² = straight-chain or branched C₂- to C₄-alkylene;
X = O;
R³, R⁴ and R⁶ = I and
R⁵ and R⁷ = H, or
R³, R⁵ and R⁷ = I and
R⁴ and R⁶ = NH(COCH₃) and
n = 1 or 2.

3. Benzoic acid derivatives according to Claim 1, characterized in that
R¹ = CH₃;
R² = -CH₂CH₂- or -CH₂CH(-)CH₂-;
X = O;
R³, R⁴ and R⁶ = I and
R⁵ and R⁷ = H, or
R³, R⁵ and R⁷ = I and
R⁴ and R⁶ = NH(COCH₃) and
n = 1 or 2.

4. X-ray opaque acrylate and/or methacrylate polymers, characterized in that they are prepared using at least one compound according to one of Claims 1 to 3 as the monomer component.

5. X-ray opaque acrylate and/or methacrylate polymers according to Claim 4, characterized in that the proportion of monomer component(s) according to one of Claims 1 to 3 in the total mass of the polymer is 20 to 60% by weight.

6. X-ray opaque acrylate and/or methacrylate polymers according to one of Claims 4 or 5, characterized in that they are prepared using one or more mono- and/or polyfunctional methacrylates as comonomers.

7. X-ray opaque acrylate and/or methacrylate polymers, characterized in that they are prepared using at least one ester or amide of iodine-substituted benzoic acid of the formula 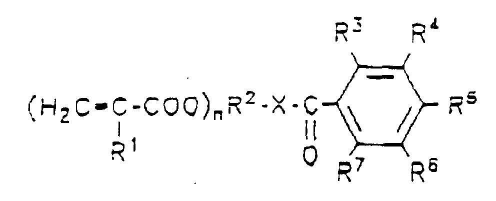 in which
R¹ = hydrogen or C₁- to C₃-alkyl;
R² = straight-chain or branched C₁- to C₆-alkylene, -oxyalkylene or -arylene;
X = O or NH;
R³-R⁷ = at least 3 iodine substituents, and the other radicals are hydrogen, C₁- to C₆-alkyl, C₁- to C₆-alkoxy, -Cl, -Br, -OH, -NH₂, -N(C₁- to C₆-alkyl)₂ or -NH-CO-(C₁- to C₆-alkyl) ; and
n = 1, 2 or 3,
as the monomer component and bisphenol A glycidyl methacrylate and/or the urethane dimethacrylate from hydroxyethyl methacrylate and 2,2,4-trimethylhexamethylene 1,6-diisocyanate as a comonomer.

8. X-ray opaque dental material, characterized in that it comprises at least one X-ray opaque monomer according to one of Claims 1 to 3 and/or X-ray opaque polymer according to one of Claims 4 to 7.

9. X-ray opaque dental material, characterized in that it comprises at least one ester or one amide of iodine-substituted benzoic acid of the formula 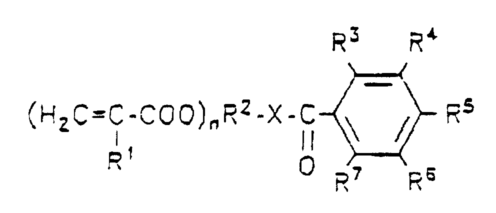 in which
R¹ = hydrogen or C₁- to C₃-alkyl;
R² = straight-chain or branched C₁- to C₆-alkylene, -oxyalkylene or -arylene;
X = O or NH;
R³-R⁷ = at least 3 iodine substituents, and the other radicals are hydrogen, C₁- to C₆-alkyl, C₁- to C₆-alkoxy, -Cl, -Br, -OH, -NH₂, -N(C₁- to C₆-alkyl) ₂ or -NH-CO-(C₁- to C₆-alkyl); and
n = 1, 2 or 3,
and/or X-ray opaque polymer according to one of Claims 4 to 7 and an initiator for photopolymerization.

10. X-ray opaque dental material according to Claim 8, characterized in that it comprises 5 to 100% by weight of a monomer or mixture of monomers according to one of Claims 1 to 3.

11. Dental material according to one of Claims 8 to 10, characterized in that it comprises
dibenzoyl peroxide and/or dilauryl peroxide as an initiator for hot polymerization, or in that it comprises
benzoyl peroxide, lauryl peroxide and N,N-dimethyl-sym.-xylidine or N,N-dimethyl-p-toluidine; or (1-methoxy-2-methyl-1-propenyloxy)trimethylsilane or bis(1-methoxy-2-methyl-1-propenoxy)methylsilane and tetrabutylammonium cyanide or tin bromide or tetrabutylammonium cyanide or tris(dimethylamino)sulphonium bifluoride as an initiator for cold polymerization; or in that it comprises
a benzophenone, benzoin and/or an α-diketone as an initiator for photopolymerization.

12. Dental material according to Claim 11, characterized in that it comprises a photoinitiator, triethylene glycol dimethacrylate and, based on the total mass of the dental material, up to 95% by weight of urethane dimethacrylate and/or bisphenol A glycidyl methacrylate.

13. Dental material according to Claim 12, characterized in that it comprises 30 to 60% by weight of urethane dimethacrylate and/or bisphenol A glycidyl methacrylate.

14. Use of dental materials according to one of Claims 8 to 13 as dental filling material, fixing cement and/or bonding.

15. Use of benzoic acid derivatives according to one of Claims 1 to 3 and/or of acrylate and/or methacrylate polymers according to one of Claims 4 to 7 for the preparation of X-ray opaque dental materials, such as filling composites, fixing cements, adhesion promoters (bondings), false teeth, inlays, implants, crowns, bridges or ready-made components.

16. Use of benzoic acid derivatives according to one of Claims 1 to 3 and/or of acrylate and/or methacrylate polymers according to one of Claims 4 to 7 for the preparation of X-ray opaque dental filling materials.

17. False teeth, veneers, implants, crowns, bridges or inlays, characterized in that they have been produced using at least one monomer according to one of Claims 1 to 3 and/or polymer according to one of Claims 4 to 7 and/or dental materials according to one of Claims 8 to 13.

## Claims (Claims for the following Contracting State(s): AT)

1. X-ray opaque esters or amides of iodine-substituted benzoic acid of the formula 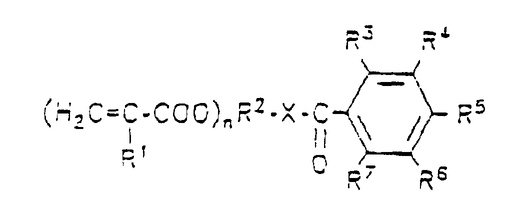 in which
R¹ = hydrogen or C₁- to C₃-alkyl;
R² = straight-chain or branched C₁- to C₆-alkylene, -oxyalkylene or -arylene;
X = O or NH;
R³-R⁷ = at least 3 iodine substituents, and the other radicals are hydrogen, C₁- to C₆-alkyl, C₁- to C₆-alkoxy, -Cl, -Br, -OH, -NH₂, -N(C₁- to C₆-alkyl)₂ or -NH-CO-(C₁- to C₆-alkyl); and
n = 1, 2 or 3,
where X can not be O when n is equal to 1, R² is ethylene or 1,2-propylene or straight-chain or branched C₄- to C₆-oxyalkylene and R¹ is hydrogen or methyl and R³-R⁷ are iodine or hydrogen.

2. Benzoic acid derivatives according to Claim 1, characterized in that
R¹ = H or CH₃;
R² = straight-chain or branched C₂- to C₄-alkylene;
X = O;
R³, R⁴ and R⁶ = I and
R⁵ and R⁷ = H, or
R³, R⁵ and R⁷ = I and
R⁴ and R⁶ = NH(COCH₃) and
n = 1 or 2.

3. Benzoic acid derivatives according to Claim 1, characterized in that
R¹ = CH₃;
R² = -CH₂CH₂- or -CH₂CH(-)CH₂-;
X = O;
R³, R⁴ and R⁶ = I and
R⁵ and R⁷ = H, or
R³, R⁵ and R⁷ = I and
R⁴ and R⁶ = NH(COCH₃) and
n = 1 or 2.

4. X-ray opaque acrylate and/or methacrylate polymers, characterized in that they are prepared using at least one compound according to one of Claims 1 to 3 as the monomer component.

5. X-ray opaque acrylate and/or methacrylate polymers according to Claim 4, characterized in that the proportion of monomer component(s) according to one of Claims 1 to 3 in the total mass of the polymer is 20 to 60% by weight.

6. X-ray opaque acrylate and/or methacrylate polymers according to one of Claims 4 or 5, characterized in that they are prepared using one or more mono- and/or polyfunctional methacrylates as comonomers.

7. X-ray opaque acrylate and/or methacrylate polymers, characterized in that they are prepared using at least one ester or amide of iodine-substituted benzoic acid of the formula 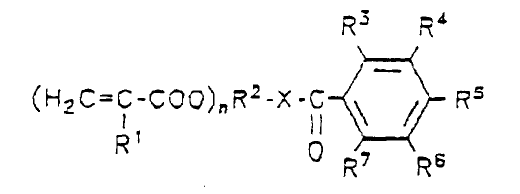 in which
R¹ = hydrogen or C₁- to C₃-alkyl;
R² = straight-chain or branched C₁- to C₆-alkylene, -oxyalkylene or -arylene;
X = O or NH;
R³-R⁷ = at least 3 iodine substituents, and the other radicals are hydrogen, C₁- to C₆-alkyl, C₁- to C₆-alkoxy, -Cl, -Br, -OH, -NH₂, -N(C₁- to C₆-alkyl)₂ or -NH-CO-(C₁- to C₆-alkyl); and
n = 1, 2 or 3,
as the monomer component and bisphenol A glycidyl methacrylate and/or the urethane dimethacrylate from hydroxyethyl methacrylate and 2,2,4-trimethylhexamethylene 1,6-diisocyanate as a comonomer.

8. X-ray opaque dental material, characterized in that it comprises at least one X-ray opaque monomer according to one of Claims 1 to 3 and/or X-ray opaque polymer according to one of Claims 4 to 7.

9. X-ray opaque dental material, characterized in that it comprises at least one ester or one amide of iodine-substituted benzoic acid of the formula 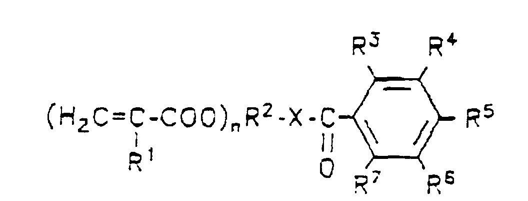 in which
R¹ = hydrogen or C₁- to C₃-alkyl;
R² = straight-chain or branched C₁- to C₆-alkylene, -oxyalkylene or -arylene;
X = O or NH;
R³-R⁷ = at least 3 iodine substituents, and the other radicals are hydrogen, C₁- to C₆-alkyl, C₁- to C₆-alkoxy, -Cl, -Br, -OH, -NH₂, -N(C₁- to C₆-alkyl)₂ or -NH-CO-(C₁- to C₆-alkyl); and
n = 1, 2 or 3,
and/or X-ray opaque polymer according to one of Claims 4 to 7 and an initiator for photopolymerization.

10. X-ray opaque dental material according to Claim 8, characterized in that it comprises 5 to 100% by weight of a monomer or mixture of monomers according to one of Claims 1 to 3.

11. Dental material according to one of Claims 8 to 10, characterized in that it comprises
dibenzoyl peroxide and/or dilauryl peroxide as an initiator for hot polymerization, or in that it comprises
benzoyl peroxide, lauryl peroxide and N,N-dimethyl-sym.-xylidine or N,N-dimethyl-p-toluidine; or (1-methoxy-2-methyl-1-propenyloxy)trimethylsilane or bis(1-methoxy-2-methyl-1-propenoxy)methylsilane and tetrabutylammonium cyanide or tin bromide or tetrabutylammonium cyanide or tris(dimethylamino)sulphonium bifluoride as an initiator for cold polymerization; or in that it comprises
a benzophenone, benzoin and/or an α-diketone as an initiator for photopolymerization.

12. Dental material according to Claim 11, characterized in that it comprises a photoinitiator, triethylene glycol dimethacrylate and, based on the total mass of the dental material, up to 95% by weight of urethane dimethacrylate and/or bisphenol A glycidyl methacrylate.

13. Dental material according to Claim 12, characterized in that it comprises 30 to 60% by weight of urethane dimethacrylate and/or bisphenol A glycidyl methacrylate.

14. Use of dental materials according to one of Claims 8 to 13 as dental filling material, fixing cement and/or bonding.

15. Use of benzoic acid derivatives according to one of Claims 1 to 3 and/or of acrylate and/or methacrylate polymers according to one of Claims 4 to 7 for the preparation of X-ray opaque dental materials, such as filling composites, fixing cements, adhesion promoters (bondings), false teeth, inlays, implants, crowns, bridges or ready-made components.

16. Use of benzoic acid derivatives according to one of Claims 1 to 3 and/or of acrylate and/or methacrylate polymers according to one of Claims 4 to 7 for the preparation of X-ray opaque dental filling materials.

17. False teeth, veneers, implants, crowns, bridges or inlays, characterized in that they have been produced using at least one monomer according to one of Claims 1 to 3 and/or polymer according to one of Claims 4 to 7 and/or dental materials according to one of Claims 8 to 13.

## Revendications (Revendications pour l'(les) Etat(s) contractant(s) suivant(s): CH, DE, FR, GB, IT, LI, SE)

1. Esters ou amides de l'acide benzoïque substitué par de l'iode, opaques aux rayons X, de formule : 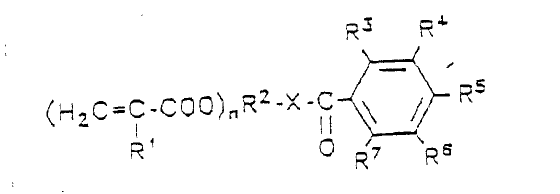
R¹ = un atome d'hydrogène ou un groupe alkyle en C₁ jusqu'à C₃,
R² = un groupe alkylène, oxyalkylène ou arylène à chaîne linéaire ou ramifiée en C₁ jusqu'à C₆;
X = un atome d'oxygène ou un groupe NH ;
R³-R⁷ = au moins trois substituants iodés, les autres radicaux sont l'atome d'hydrogène, les groupes alkyle en C₁ jusqu'à C₆, alcoxy en C₁ jusqu'à C₆, Cl-, Br-, -OH, -NH₂, -N-(alkyle en C₁ jusqu'à C₆)₂ ou -NH-CO-(alkyle en C₁ jusqu'à C₆) ; et
n = 1, 2 ou 3 ;
étant entendu que X n'est pas O lorsque n est 1, R² représente C₁-C₄ alkylène ou C₄-C₆ oxyalkylène, linéaire ou ramifié, et R¹ représente un atome d'hydrogène ou un groupe méthyle et R³-R⁷ sont l'iode, le brome ou un atome d'hydrogène.

2. Dérivés de l'acide benzoïque selon la revendication 1, caractérisés en ce que
R¹ = un atome d'hydrogène ou un groupe CH₃;
R² = un groupe alkylène à chaîne linéaire ou ramifiée en C₂ jusqu'à C₄ ;
X = un atome d'oxygène;
R³, R⁴ et R⁶ = un atome d'iode et
R⁵ et R⁷ = un atome d'hydrogène, ou
R³, R⁵ et R⁷ = un atome d'iode et
R⁴ et R⁶ = NH(COCH₃) et
n = 1 ou 2.

3. Dérivés de l'acide benzoïque selon la revendication 1, caractérisés en ce que,
R¹ = un groupe CH₃;
R² = un groupe -CH₂-CH₂- ou -CH₂-CH(-)CH₂-;
X = un atome d'oxygène;
R³, R⁴ et R⁶ = un atome d'iode et
R⁵ et R⁷ = un atome d'hydrogène, ou
R³, R⁵ et R⁷ = un atome d'iode et
R⁴ et R⁶ = NH(COCH₃) et
n = 1 ou 2.

4. Polymères acrylate et/ou méthacrylate opaques aux rayons X, caractérisés en ce qu'ils sont préparés en utilisant au moins un composé selon une des revendications 1 à 3 comme composant monomère.

5. Polymères acrylate et/ou méthacrylate opaques aux rayons X, selon la revendication 4, caractérisés en ce que la teneur en composant(s) monomère(s) selon une des revendications 1 à 3 est comprise entre 20 et 60% en poids par rapport à la masse totale du polymère.

6. Polymères acrylate et/ou méthacrylate opaques aux rayons X, selon une des revendications 4 ou 5, caractérisés en ce qu'ils sont préparés en utilisant un ou plusieurs méthacrylates mono-et/ou poly-fonctionnels comme comonomères.

7. Polymères méthacrylate et/ou acrylate opaques aux rayons X, caractérisés en ce qu'ils sont préparés par utilisation d'au moins un ester ou amide de l'acide benzoïque substitué par de l'iode, de formule : 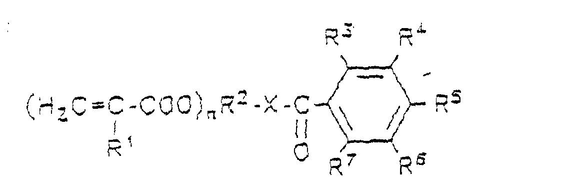 dans laquelle :
R¹ = un atome d'hydrogène ou un groupe alkyle en C₁ jusqu'à C₃,
R² = un groupe alkylène, oxyalkylène ou arylène à chaîne linéaire ou ramifiée en C₁ jusqu'à C₆;
X = un atome d'oxygène ou un groupe NH ;
R³-R⁷ = au moins trois substituants iodés, les autres radicaux sont l'atome d'hydrogène, les groupes alkyle en C₁ jusqu'à C₆, alcoxy en C₁ jusqu'à C₆, Cl-, Br-, -OH, -NH₂, -N-(alkyle en C₁ jusqu'à C₆)₂ ou -NH-CO-(alkyle en C₁ jusqu'à C₆); et
n = 1, 2 ou 3 ;
en tant que composants monomères et de méthacrylate de Bisphénol-A-glycidyle et/ou de diméthacrylate d'uréthanne dérivé d'hydroxyéthylméthacrylate et de 2,2,4-triméthylhexaméthylènediisocyanate-1,6 en tant que comonomère.

8. Matériau dentaire opaque aux rayons X, caractérisé en ce qu'il contient au moins un monomère opaque aux rayons X selon une des revendications 1 à 3 et/ou un polymère opaque aux rayons X selon une des revendications 4 à 7.

9. Matériau dentaire opaque aux rayons X, caractérisé en ce qu'il contient au moins un ester ou amide de l'acide benzoïque substitué par de l'iode de formule : 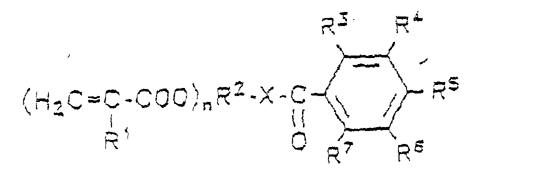 dans laquelle :
R¹ = un atome d'hydrogène ou un groupe alkyle en C₁ jusqu'à C₃,
R² = un groupe alkylène, oxyalkylène ou arylène à chaîne linéaire ou ramifiée en C₁ jusqu'à C₆ ;
X = un atome d'oxygène ou un groupe NH ;
R³-R⁷ = au moins trois substituants iodés, les autres radicaux sont l'atome d'hydrogène, les groupes alkyle en C₁ jusqu'à C₆, alcoxy en C₁ jusqu'à C₆, Cl-, Br-, -OH, -NH₂, -N-(alkyle en C₁ jusqu'à C₆)₂ ou -NH-CO-(alkyle en C₁ jusqu'à C₆) ; et
n = 1, 2 ou 3 ;
et/ou un polymère opaque aux rayons X selon l'une des revendications 4 à 7 et un initiateur pour la photopolymérisation.

10. Matériau dentaire opaque aux rayons X selon la revendication 8, caractérisé en ce qu'il contient de 5 à 100% en poids d'un monomère ou d'un mélange de monomères selon une des revendications 1 à 3.

11. Matériau dentaire selon une des revendications 8 à 10, caractérisé en ce qu'il contient comme initiateur pour la polymérisation à chaud du peroxyde de dibenzoyle et/ou du peroxyde de dilauryle ou
en ce qu'il contient comme initiateur pour la polymérisation à froid, du peroxyde de benzoyle, du peroxyde de lauryle et de la N,N-diméthyl-sym.-xylidine ou de la N,N-diméthyl-p-toluidine; ou du (1-méthoxy-2-méthyl-1-propényl-oxy)triméthylsilane ou du bis(1-méthoxy-2-méthyl-1-propènoxy)méthylsilane et du cyanure de tétrabutylammonium ou du bromure de zinc ou du bifluorure de tris(diméthylamino)sulfonium; ou
en ce qu'il contient comme initiateur pour la photopolymérisation une benzophénone, de la benzoïne et/ou une α-dicétone.

12. Matériau dentaire selon la revendication 11, caractérisé en ce qu'il contient un photoinitiateur, du diméthacrylate de triéthylèneglycol et jusqu'à 95% en poids de diméthacrylate d'uréthanne et/ou de méthacrylate de bisphénol-A-glycidyle par rapport à la masse totale du matériau dentaire.

13. Matériau dentaire selon la revendication 12, caractérisé en ce qu'il contient de 30 à 60% en poids de diméthacrylate d'uréthanne et/ou de méthacrylate de bisphénol-A-glycidyle.

14. Utilisation de matériaux dentaires selon une des revendications 8 à 13 comme matériau de remplissage dentaire, ciment de consolidation et/ou de bonding.

15. Utilisation de dérivés de l'acide benzoïque selon une des revendications 1 à 3 et/ou de polymères d'acrylate et/ou de méthacrylate selon une des revendications 4 à 7 pour la fabrication de matériaux dentaires opaques aux rayons X, tels que des matériaux composites de remplissage, des ciments de consolidation, des agents adhésifs (bondings), des prothèses dentaires, des inlays, des implants, des couronnes, des bridges ou des pièces finies.

16. Utilisation de dérivés de l'acide benzoïque selon une des revendications 1 à 3 et/ou de polymères d'acrylate et/ou de méthacrylate selon une des revendications 4 à 7 pour la préparation de matériaux dentaires de remplissage opaques aux rayons X.

17. Prothèses dentaires, couches de plaquage (veneers), implants, couronnes, bridges ou inlays, caractérisé en ce qu'ils ont été préparés en utilisant au moins un monomère selon une des revendications 1 à 3 et/ou un polymère selon une des revendications 4 à 7 et/ou des matériaux dentaires selon une des revendications 8 à 13.

## Revendications (Revendications pour l'(les) Etat(s) contractant(s) suivant(s): AT)

1. Esters ou amides de l'acide benzoïque substitué par de l'iode, opaques aux rayons X, de formule : 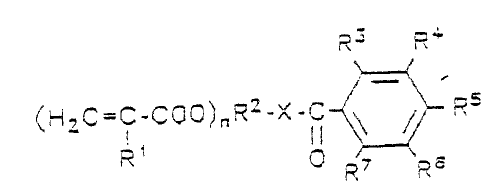
R¹ = un atome d'hydrogène ou un groupe alkyle en C₁ jusqu'à C₃,
R² = un groupe alkylène, oxyalkylène ou arylène à chaîne linéaire ou ramifiée en C₁ jusqu'à C₆;
X = un atome d'oxygène ou un groupe NH ;
R³-R⁷ = au moins trois substituants iodés, les autres radicaux sont l'atome d'hydrogène, les groupes alkyle en C₁ jusqu'à C₆, alcoxy en C₁ jusqu'à C₆, Cl-, Br-, -OH, -NH₂, -N-(alkyle en C₁ jusqu'à C₆)₂ ou -NH-CO-(alkyle en C₁ jusqu'à C₆); et
n = 1, 2 ou 3 ;
étant entendu que X n'est pas O lorsque n est 1, R² représente éthylène ou 1,2-propylène ou C₄-C₆ oxyalkylène, linéaire ou ramifié, et R¹ représente un atome d'hydrogène ou un groupe méthyle et R³-R⁷ sont l'iode, le brome ou un atome d'hydrogène.

2. Dérivés de l'acide benzoïque selon la revendication 1, caractérisés en ce que
R¹ = un atome d'hydrogène ou un groupe CH₃;
R² = un groupe alkylène à chaîne linéaire ou ramifiée en C₂ jusqu'à C₄ ;
X = un atome d'oxygène;
R³, R⁴ et R⁶ = un atome d'iode et
R⁵ et R⁷ = un atome d'hydrogène, ou
R³, R⁵ et R⁷ = un atome d'iode et
R⁴ et R⁶ = NH(COCH₃) et
n = 1 ou 2.

3. Dérivés de l'acide benzoïque selon la revendication 1, caractérisés en ce que,
R¹ = un groupe CH₃;
R² = un groupe -CH₂-CH₂- ou -CH₂-CH (-) CH₂-;
X = un atome d'oxygène;
R³, R⁴ et R⁶ = un atome d'iode et
R⁵ et R⁷ = un atome d'hydrogène, ou
R³, R⁵ et R⁷ = un atome d'iode et
R⁴ et R⁶ = NH(COCH₃) et
n = 1 ou 2.

4. Polymères acrylate et/ou méthacrylate opaques aux rayons X, caractérisés en ce qu'ils sont préparés en utilisant au moins un composé selon une des revendications 1 à 3 comme composant monomère.

5. Polymères acrylate et/ou méthacrylate opaques aux rayons X, selon la revendication 4, caractérisés en ce que la teneur en composant (s) monomère (s) selon une des revendications 1 à 3 est comprise entre 20 et 60% en poids par rapport à la masse totale du polymère.

6. Polymères acrylate et/ou méthacrylate opaques aux rayons X, selon une des revendications 4 ou 5, caractérisés en ce qu'ils sont préparés en utilisant un ou plusieurs méthacrylates mono-et/ou poly-fonctionnels comme comonomères.

7. Polymères méthacrylate et/ou acrylate opaques aux rayons X, caractérisés en ce qu'ils sont préparés par utilisation d'au moins un ester ou amide de l'acide benzoïque substitué par de l'iode, de formule : 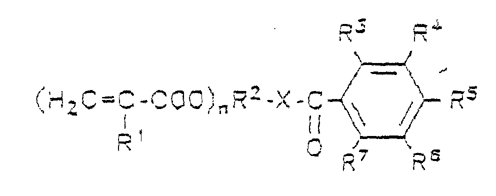 dans laquelle :
R¹ = un atome d'hydrogène ou un groupe alkyle en C₁ jusqu'à C₃,
R² = un groupe alkylène, oxyalkylène ou arylène à chaîne linéaire ou ramifiée en C₁ jusqu'à C₆;
X = un atome d'oxygène ou un groupe NH;
R³-R⁷ = au moins trois substituants iodés, les autres radicaux sont l'atome d'hydrogène, les groupes alkyle en C₁ jusqu'à C₆, alcoxy en C₁ jusqu'à C₆, Cl-, Br-, -OH, -NH₂, -N-(alkyle en C₁ jusqu'à C₆)₂ ou -NH-CO-(alkyle en C₁ jusqu'à C₆); et
n = 1, 2 ou 3 ;
en tant que composants monomères et de méthacrylate de Bisphénol-A-glycidyle et/ou de diméthacrylate d'uréthanne dérivé d'hydroxyéthylméthacrylate et de 2,2,4-triméthylhexaméthylènediisocyanate-1,6 en tant que comonomère.

8. Matériau dentaire opaque aux rayons X, caractérisé en ce qu'il contient au moins un monomère opaque aux rayons X selon une des revendications 1 à 3 et/ou un polymère opaque aux rayons X selon une des revendications 4 à 7.

9. Matériau dentaire opaque aux rayons X, caractérisé en ce qu'il contient au moins un ester ou amide de l'acide benzoïque substitué par de l'iode de formule : 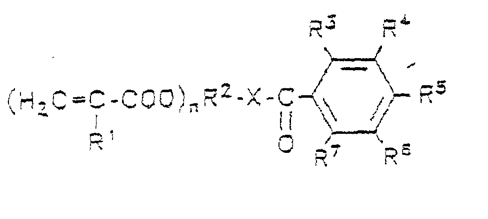 dans laquelle :
R¹ = un atome d'hydrogène ou un groupe alkyle en C₁ jusqu'à C₃,
R² = un groupe alkylène, oxyalkylène ou arylène à chaîne linéaire ou ramifiée en C₁ jusqu'à C₆;
X = un atome d'oxygène ou un groupe NH ;
R³-R⁷ = au moins trois substituants iodés, les autres radicaux sont l'atome d'hydrogène, les groupes alkyle en C₁ jusqu'à C₆, alcoxy en C₁ jusqu'à C₆, Cl-, Br-, -OH, -NH₂, -N-(alkyle en C₁ jusqu'à C₆)₂ ou -NH-CO-(alkyle en C₁ jusqu'à C₆); et
n = 1, 2 ou 3 ;
et/ou un polymère opaque aux rayons X selon l'une des revendications 4 à 7 et un initiateur pour la photopolymérisation.

10. Matériau dentaire opaque aux rayons X selon la revendication 8, caractérisé en ce qu'il contient de 5 à 100% en poids d'un monomère ou d'un mélange de monomères selon une des revendications 1 à 3.

11. Matériau dentaire selon une des revendications 8 à 10, caractérisé en ce qu'il contient comme initiateur pour la polymérisation à chaud du peroxyde de dibenzoyle et/ou du peroxyde de dilauryle ou
en ce qu'il contient comme initiateur pour la polymérisation à froid, du peroxyde de benzoyle, du peroxyde de lauryle et de la N,N-diméthyl-sym.-xylidine ou de la N,N-diméthyl-p-toluidine; ou du (1-méthoxy-2-méthyl-1-propényl-oxy)triméthylsilane ou du bis(1-méthoxy-2-méthyl-1-propènoxy)méthylsilane et du cyanure de tétrabutylammonium ou du bromure de zinc ou du bifluorure de tris(diméthylamino)sulfonium; ou
en ce qu'il contient comme initiateur pour la photopolymérisation une benzophénone, de la benzoïne et/ou une α-dicétone.

12. Matériau dentaire selon la revendication 11, caractérisé en ce qu'il contient un photoinitiateur, du diméthacrylate de triéthylèneglycol et jusqu'à 95% en poids de diméthacrylate d'uréthanne et/ou de méthacrylate de bisphénol-A-glycidyle par rapport à la masse totale du matériau dentaire.

13. Matériau dentaire selon la revendication 12, caractérisé en ce qu'il contient de 30 à 60% en poids de diméthacrylate d'uréthanne et/ou de méthacrylate de bisphénol-A-glycidyle.

14. Utilisation de matériaux dentaires selon une des revendications 8 à 13 comme matériau de remplissage dentaire, ciment de consolidation et/ou de bonding.

15. Utilisation de dérivés de l'acide benzoïque selon une des revendications 1 à 3 et/ou de polymères d'acrylate et/ou de méthacrylate selon une des revendications 4 à 7 pour la fabrication de matériaux dentaires opaques aux rayons X, tels que des matériaux composites de remplissage, des ciments de consolidation, des agents adhésifs (bondings), des prothèses dentaires, des inlays, des implants, des couronnes, des bridges ou des pièces finies.

16. Utilisation de dérivés de l'acide benzoïque selon une des revendications 1 à 3 et/ou de polymères d'acrylate et/ou de méthacrylate selon une des revendications 4 à 7 pour la préparation de matériaux dentaires de remplissage opaques aux rayons X.

17. Prothèses dentaires, couches de plaquage (veneers), implants, couronnes, bridges ou inlays, caractérisé en ce qu'ils ont été préparés en utilisant au moins un monomère selon une des revendications 1 à 3 et/ou un polymère selon une des revendications 4 à 7 et/ou des matériaux dentaires selon une des revendications 8 à 13.
